# EUROPEAN PATENT SPECIFICATION

(11) **EP 1 912 927 B1**
(45) Date of publication and mention of the grant of the patent: **17.08.2016**
(21) Application number: 06788703.4
(22) Date of filing: 27.07.2006
(51) Int. Cl.: C07C 51/44, C07C 63/26, C07C 51/47

(54) **PROCESS FOR REMOVAL OF BENZOIC ACID FROM AN OXIDIZER PURGE STREAM**
VERFAHREN ZUM ENTFERNEN VON BENZOESÄURE AUS EINEM OXIDIZER-REINIGUNGSSTROM
PROCEDE D'ELIMINATION D'ACIDE BENZOIQUE PROVENANT D'UN FLUX DE PURGE D'UN OXYDANT

(30) Priority: 11.08.2005 US 201512; 11.08.2005 US 201799
(43) Date of publication of application: 23.04.2008
(73) Proprietor: GRUPO PETROTEMEX, S.A. DE C.V., San Pedro Garza Garcia, Nuevo Leon 66265 (MX)
(72) Inventor: LIN, Robert, Kingsport, TN 37664 (US); GIBSON, Philip, Edward, Kingsport, TN 37660 (US); PARKER, Kenny, Randolph, Afton, TN 37616 (US)
(74) Representative: Tostmann, Holger Carl
(86) International application number: PCT/US2006/029267
(87) International publication number: WO 2007/021487

(56) References cited:
- WO-A-97/30963
- GB-A- 892 766

## Description

### FIELD OF INVENTION

This invention relates to the removal of impurities, specifically benzoic acid, from a mother liquor produced in the synthesis of carboxylic acid, typically terephthalic acid. This invention also relates to the removal of impurities, specifically benzoic acid, from a benzoic acid bearing stream produced in the synthesis of carboxylic acid.

### BACKGROUND OF THE INVENTION

Terephthalic acid is commercially produced by oxidation of paraxylene in the presence of a catalyst, such as, for example, Co, Mn, Br and a solvent. Terephthalic acid used in the production of polyester fibers, films, and resins must be further treated to remove impurities formed as a result of the oxidation of paraxylene.

Terephthalic acid (TPA) is an intermediate in the production of polyesters for plastics and fiber applications. Commercial processes for the manufacture of TPA are often based on the heavy-metal catalyzed oxidation of p-xylene, generally with a bromide promoter in an acetic acid solvent. Due to the limited solubility of TPA in acetic acid under practical oxidation conditions, a slurry of TPA crystals is usually formed in the oxidation reactor. Typically, the TPA oxidizer slurry is withdrawn from the reactor, and TPA solids are separated from the oxidizer mother liquor using conventional solid-liquid separation techniques. The oxidizer mother liquor, which contains most of the catalyst and promoter used in the process, is recycled to the oxidation reactor. Aside from the catalyst and promoter, the oxidizer mother liquor stream also contains dissolved TPA and many by-products and impurities. These by-products and impurities arise partially from minor impurities present in the p-xylene feed stream. Other impurities arise due to the incomplete oxidation of p-xylene resulting in partially oxidized products. Still other by-products result from competing side reactions formed as a result of the oxidation of p-xylene to terephthalic acid.

The TPA solids undergo a solid-liquid separation wherein fresh solvent is utilitized to displace a major portion of the liquid component of the oxidizer mother liquor. After drying, the TPA solids are contaminated with impurities that were present in the oxidizer mother liquor since these impurities may be incorporated into the TPA solids. Impurities are also present due to occlusions in the TPA crystal structure and due to incomplete removal of the oxidizer mother liquor by the fresh solvent wash.

Many of the impurities in the oxidizer mother liquor stream that are recycled are relatively inert to further oxidation. Such impurities include, for example, isophthalic acid, phthalic acid and trimellitic acid. Impurities, which may undergo further oxidation are also present, such as, for example, 4-carboxybenzaldehyde, p-toluic acid and p-tolualdehyde. Oxidation inert impurities tend to accumulate in the oxidizer mother liquor upon recycle. The concentration of these inert impurities will increase in the oxidizer mother liquor until an equilibria is reached whereby the rate of removal of each impurity via the TPA product balances with the rate of formation and the rate of addition to the oxidation process. The normal level of impurities in commercial crude TPA makes it unsuitable for direct use In most polymer applications.

Conventionally, crude TPA has been purified either by conversion of a dimethyl ester or by dissolution In water with subsequent hydrogenation over standard hydrogenation catalysts. More recently, secondary oxidative treatments have been used to produce polymer-grade TPA. It is desirable to minimize the concentration of impurities in the mother liquor and thereby facilitate subsequent purification of TPA. In some cases, it is not possible to produce a purified, polymer-grade TPA unless some means for removing impurities from the oxidizer mother liquor stream are utilized.

GB 892,766 describes improvements relating to the production of aromatic acids, in particular the recovering of oxidation catalysts employed for the liquid phase oxidation of aliphatic substituted aromatic compounds or fused ring aromatic compounds, to aromatic carboxylic acids. The process comprises extracting the bottoms fraction in molten or solid state with a solvent mixture selected from water and lower saturated paraffinic and naphthanic monocarboxylic acids to separate the oxidation catalysts from undissolved oxidation by-products.

WO 97/30963 relates to a mother liquor that is derived from a primary solids-liquid separation process used for separating aromatic carboxylic acid crystals from a slurry thereof, in the mother liquor. Specifically, the mother liquor is split into a recycle fraction and a purge fraction, wherein the recycle fraction is returned to a reactor, in which the aromatic carboxylic acid is formed.

One technique for impurity removal from a recycle stream commonly used In the chemical processing industry is to draw out or "purge" some portion of the recycle stream. Typically, the purge stream is simply disposed of or, if economically justified, subjected to various treatments to remove undesired impurities while recovering valuable components. One example of this process is U.S. # 4,939,297. The amount of purge required for control of impurities Is process-dependent; however, a purge amount equal to 10-40 wt% of the total oxidizer mother liquor stream is usually sufficient to produce TPA adequate as feedstock for commercial polymer manufacture. In the production of TPA, the percentage of the oxidizer mother liquor stream purge necessary to maintain acceptable impurity concentrations, coupled with the economic value of the metal catalyst and solvent components in the oxidizer purge stream, make simple disposal of the oxidizer purge stream economically unattractive. Thus, there is a need for a process that recovers essentially all of the valuable metal catalysts and acetic acid contained in the oxidizer purge stream while removing a major portion of the impurities present in the oxidizer purge stream. The metal catalyst can be recovered in an active form suitable for reuse by direct recycling to the p-xylene oxidation step.

One benefit of this invention is the energy and capital cost savings compared with the extraction based purge process previously described.

Another benefit of this invention is its efficacy compared with extraction purge processes regarding the usefulness of the solvent stream(s) recycled to the TPA process. The primary motivation in a liquid extraction process is based upon the assumption that introducing any aromatic impurities into a p-xylene oxidation process for producing terephthalic acid has a detrimental effect on the terephthalic acid powder quality (e.g. yellow color). Hence, it was assumed that a broad spectrum removal of aromatic impurities, such as provided by liquid extraction, was necessary to achieve appropriate terephthalic acid powder quality.

In one embodiment of this invention, however, employs a relatively simple process that separates benzoic acid from an aqueous solvent. The efficiency of the process toward benzoic acid is high since benzoic acid is more volatile (a higher vapor pressure) than most Identified aromatic impurities in the production of a carboxylic acid, typically terephthalic acid. These aromatic impurities include, but are not limited to, trimellitic acid, isophthalic acid, stilbenes, and anthraquinones. Therefore, it is rather surprising that removal of a benzoic acid in favor of the other known impurities, that are inherently colored, would be sufficient to produce a carboxylic acid, typically terephthalic acid of good quality.

### SUMMARY OF THE INVENTION

The invention relates to a process as defined in claim 1.

Preferred embodiments are defined in claims 2 to 14 depending thereon.

### BRIEF DESCRIPTION OF THE DRAWINGS

Figure 1 illustrates a process to recover benzoic acid from an oxidizer purge stream **101.**
Figure 2 illustrates an embodiment of the process occurring in the solid-liquid separation zone **151** wherein the solid-liquid separation zone comprises a filtration zone **153,** a washing zone **155,** and optionally a dewatering zone **157.**
Figure 3 illustrates an embodiment where a rotary pressure drum filter is utilized in the solid-liquid separation zone.
Figure 4 illustrates an embodiment wherein a process to recover benzoic acid from an oxidizer purge stream 101 is shown, and the process utilizes a main evaporator zone **125**.
Figure 5 illustrates the invention wherein a process to recovery benzoic acid from a benzoic acid bearing stream **347** is provided.

### DESCRIPTION OF THE INVENTION:

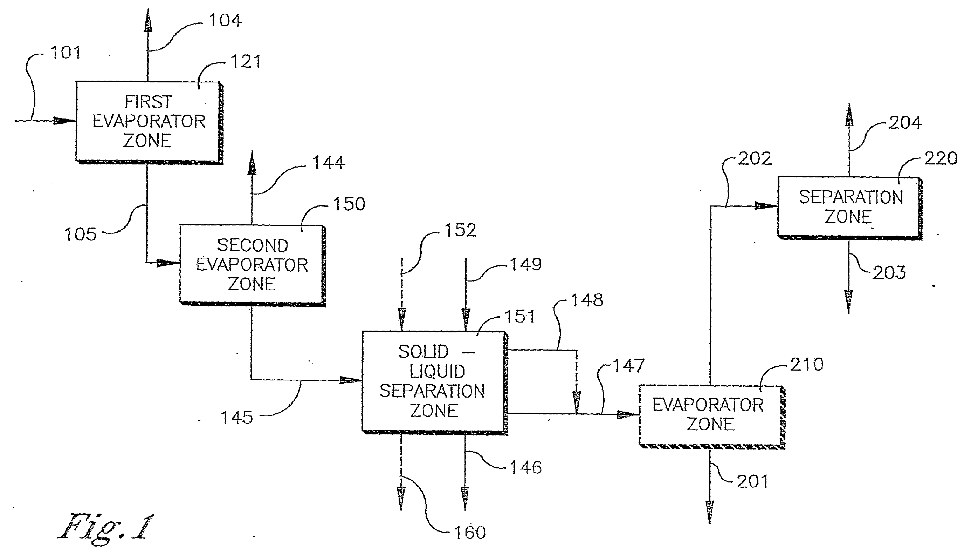

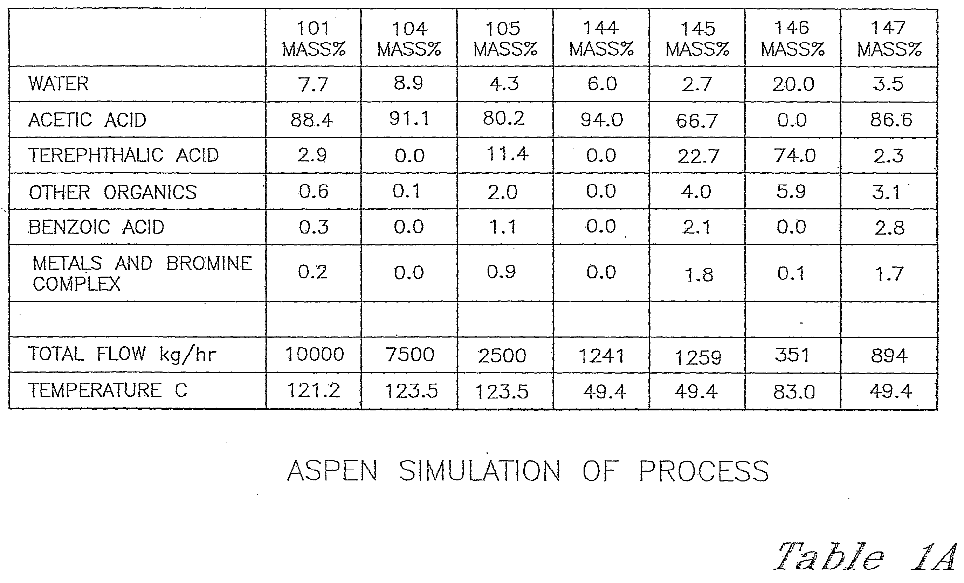

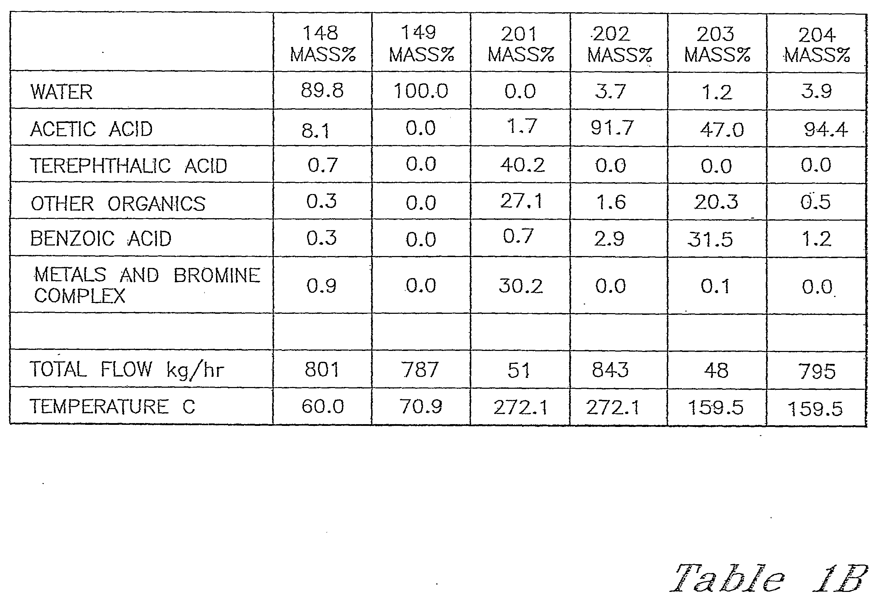

Disclosed is a process to separate benzoic acid from an oxidizer purge stream **101** as shown in Figure 1.

Step (a) comprises subjecting an oxidizer purge stream **101** to evaporation in a first evaporator zone **121** to produce a vapor stream **104** and a concentrated purge slurry **105.**

In an embodiment, the oxidizer purge stream **101** is withdrawn from a carboxylic acid oxidative synthesis process. The oxidizer purge stream **101** serves as the feed stream to the present process. In an embodiment, the oxidizer purge stream **101** comprises at least one carboxylic acid, at least one solvent, at least one metal catalyst and impurities. The impurities comprise at least one impurity selected from the group consisting of organic bromides, corrosion metals, p-xylene oxidation by-products, and impurities derived as a result of impurities in the p-xylene, The organic bromides may be used as promoters in the oxidation reaction. Examples of corrosion metals are iron and chromium compounds, which inhibit, reduce or entirely destroy the activity of the metal catalyst. Aside from the catalyst and promoter, the oxidizer purge stream **101** also contains by-products and impurities. These by-products and impurities arise partially from minor impurities present in the p-xylene feed stream. Other impurities arise due to the incomplete oxidation of p-xylene resulting In partially oxidized products. Still other by-products result from competing side reactions in the oxidation of p-xylene to terephthalic acid.

The carboxylic acids include any aromatic carboxylic acids produced via controlled oxidation of an organic substrate. Such aromatic carboxylic acids include compounds with at least one carboxylic acid group attached to a carbon atom that is part of an aromatic ring, preferably having at least 6 carbon atoms, even more preferably having only carbon atoms. Suitable examples of such aromatic rings include, but are not limited to, benzene, biphenyl, terphenyl, naphthalene, and other carbon-based fused aromatic rings. Examples of suitable carboxylic acids include, but are not limited to, terephthalic acid, p-toluic acid, isophthalic acid, trimellitic acid, naphthalene dicarboxylic acid, 2,5-diphenyl-terephthalic acid, and mixtures thereof.

Suitable solvents include, aliphatic mono-carboxylic acids, preferably containing 2 to 6 carbon atoms, or benzoic acid and mixtures thereof and mixtures of these compounds with water. Preferably, the solvent Is acetic acid mixed with water in a ratio of about 5:1 to about 25:1, preferably between about 8:1 and about 20:1. Throughout the specification, acetic acid will be referred to as the solvent. However, it should be appreciated that other suitable solvents, such as those disclosed previously, may also be utilized.

In the first step of the present process, the oxidizer purge stream **101** Is concentrated by conventional means in a first evaporator zone **121** comprising at least one evaporator to produce a vapor stream **104** and a concentrated purge slurry **105.** In an embodiment the evaporator is operated at atmospheric or slightly super atmospheric conditions, generally from 1x100000 Pa (1 atmosphere) to 1x000000 Pa (10 atmospheres). The vapor stream **104** comprises a majority of the water and solvent, and the concentrated purge slurry **105** comprises the remainder of the water and solvent not removed from the oxidizer purge stream **101.** As used herein "majority" means greater than 50% by weight. In an embodiment, the evaporation removes about 50 wt%(weight percent) to about 80 wt% of the solvent and water, typically acetic acid and water, which are present in the oxidizer purge stream **101.**

Step (b) comprising subjecting the concentrated purge slurry **105** to evaporation in a second evaporator zone **150** to produce a solvent rich stream **144** and a super concentrated purge slurry **145.**

In an embodiment, the second evaporator zone **150** comprises at least one evaporator operated at vacuum conditions. In an embodiment of the invention, the evaporation can be conducted at a temperature from about 20°C to about 70°C; another range is from about 30°C to about 50°C. In an embodiment, the combination of evaporators **121** and **150** is operated so as to concentrate the oxidizer purge stream as represented by stream **101** to a condition wherein about 75 wt% to about 99 wt% of the solvent and water, typically acetic acid and water, are removed from the oxidizer purge stream **101** to produce the super concentrated purge slurry **145.** In another embodiment another range for operation of the combination of evaporators **121** and **150** is operated so as to concentrate the oxidizer purge stream as represented by stream **101** to a condition wherein about 85 wt% to about 99 wt% of the solvent and water, typically acetic acid and water, is removed from the oxidizer purge stream **101** to produce the super concentrated purge slurry **145.**

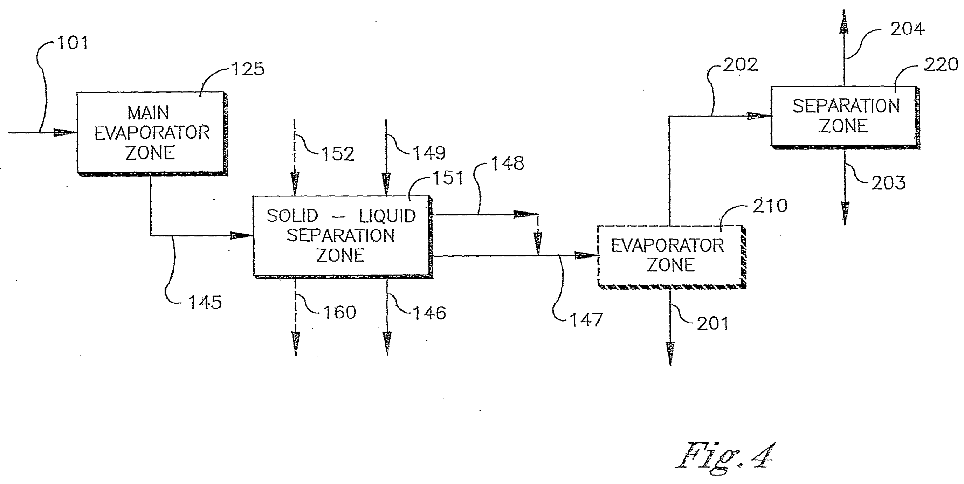

In another embodiment, the first evaporation zone **121** and the second evaporator zone can be combined in a main evaporation zone **125** as shown in Figure 4. The main evaporation zone **125** comprises at least one evaporator. The evaporator or evaporators in the main evaporation zone **125** are operated at a temperature and pressure sufficient to remove at least 75% by weight of the solvent and water combined from the oxidizer purge stream **101.** In another embodiment, the evaporator or evaporators in the main evaporation zone **125** are operated at a temperature and pressure sufficient to remove at least 85% by weight of the solvent and water combined from the oxidizer purge stream. In another embodiment, the evaporator or evaporators in the main evaporation zone **125** are operated at a temperature and pressure sufficient to remove at least 90% by weight of the solvent and water combined from the oxidizer purge stream **101.** In another embodiment, the evaporator or evaporators in the main evaporation zone **125** are operated at a temperature and pressure sufficient to remove at least 95% by weight of the solvent and water combined from the oxidizer purge stream.

Ranges stated in this disclosure and the claims that follow should be understood to disclose the entire range specifically and not just the end point(s). For example, disclosure of the range 0 to 10 should be taken to specifically disclose 2, 2.5, 3.17 and all other number subsumed and not just 0 and 10.

In an embodiment, the condition of the super concentrated purge slurry **145** can be as a solid-liquid mixture with only enough solvent to provide pumpability.

Step (c) comprises filtering the super concentrated purge slurry **145** in a solid-liquid separation zone **151** to form a filter cake **154** and a mother liquor **147.**

Step (d) comprises washing the filter cake **154** with a wash feed **149** in the solid-liquid separation zone **151** to form a washed cake **146** and a wash filtrate **148;** and optionally dewatering the washed cake **146** in the solid-liquid separation zone **151** to form a dewatered cake **159;** wherein the solid-liquid separation zone **151** comprises at least one pressure filtration device.

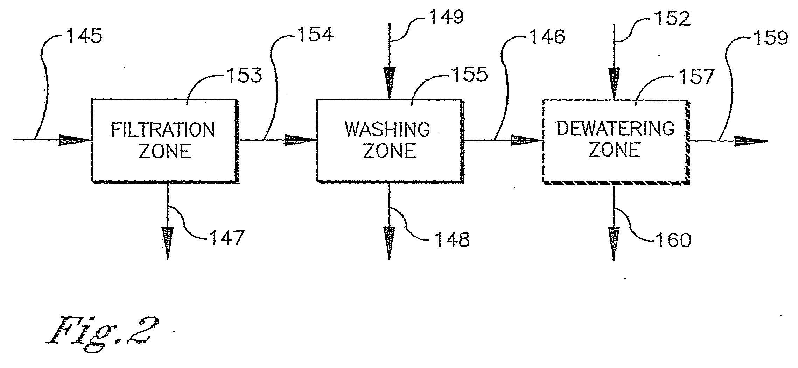

In an embodiment, the super concentrated purge slurry **145 is** introduced In the solid-liquid separation zone **151** where the solid-liquid separation zone comprises a filtration zone **153,** a washing zone **155,** and optionally a drying zone **157** as shown in Figure 2. The filtration zone **153** comprises a filter cell, or a series of filter cells, physically situated to permit a filter cake **154** to develop a distribution across the area of the filter cell to hinder or prevent the channeling of wash feed **149** through the filter cake **154.**

Suitably, a filter cake **154** of at least 0.64 cm (0.25 inch) in depth to 20 cm (8 inches) in depth, preferably at least 1.3 cm (0.5 inch) in depth, more preferably at least 2,5 cm (1 inch) in depth, and even more preferably 5 cm (2 inches) to 10 cm (4 inches) in depth is distributed over the area of the filter cell. The washed cake, **146,** can be recovered or further treated, recycled and/or sent to waste treatment facilities.

Upon obtaining a suitable or preferred height of filter cake **154** the filter cake **154** leaves the filtration zone **153** which comprises a filter or series of filters and enters a washing zone **155** where the filter cake **154** is contacted with a wash feed **149.** In one embodiment there is sufficient pressure across the filter cake **154** to allow a reservoir or buildup of the wash feed **149** over the filter cake **154** to a suitable depth, preferably to a minimum depth of 0.64 cm (0,25 inch). A pressure gradient of at least 3.4 kPa (0.5 psi), preferably from 3x10 kPa (5 psi) to 45x10 kPa (65 psi), across the filter cake **154** and the reservoir of wash feed **149** can be applied to displace any solute in the filter cake **154** with wash feed **149.**

A filter cake **154** depth of at least 1,3 cm (0,5 inch) is suitable to obtain a filter cake **154** of sufficient compactness to furnish a wash vehicle, i.e. the filter cake **154,** from which a wash filtrate **148** containing a solute from the filter cake **154** can be removed efficiently by displacement washing. If the filter cake depth **154** is less than 0.64 cm (0.25 inch), channeling of wash feed **149** in the filter cake **154** can occur resulting In non-uniform washing of the filter cake **154.**

Because of the loss of efficiency in displacement washing of the filter cake **154,** a minimum filter cake **154** depth of at least 0.64 cm (0,25 inch) of purified terephthalic acid is preferred.

A minimum liquid height above the filter cake **154** surface is required to ensure that displacement washing occurs. This height must be sufficient to ensure that the filter cake **154** surface is completely covered with wash feed **149.** If the filter cake **154** surface is not covered with wash feed **149,** bypassing of the wash feed **149** can occur without adequate displacement of the solute in the filter cake **154.** Because of Irregularities in the filter cake **154** surface, a minimum liquid height of 0.64 cm (0.25 inch) is preferred above the filter cake **154** surface.

It has been found that displacement of the solute from the filter cake **154** using the wash feed **149** at high pressure permits an efficient separation of catalyst metals from the filter cake **154.** Another benefit of high pressure Is the reduction of wash feed **149** required to recover cobalt as shown in the examples.

Utilization of added stages In the solid-liquid separation zone **151** can decrease the amount of wash feed **149** required to reduce the total amount of metal catalyst retained In the filter cake **154.** It Is convenient therefore that a suitable number of stages of positive displacement washing be used to minimize total wash feed **149** used In displacement washing to reduce need for downstream waste treatment facilities,

It is understood that multiple stages of the displacement washing procedure can replace a single stage displacement washing procedure wherein the quantity of wash feed **149** is sufficient to obtain at least 80 wt% recovery of the metal catalyst from the super concentrated slurry **145** to the mother liquor **147** and the wash filtrate **148.** Additionally, a procedure utilizing multiple stages of counter-current washing can be useful If reduction of the amount of wash feed **149** Is determined to be advantageous.

In the process, a super concentrated purge slurry **145** is Introduced into one or more of a series of filter cells physically situated to permit a filter cake **154** of requisite thickness to develop. Upon obtaining a minimum height of filter cake **154,** 0.64 cm (0,25 inch) to 10 cm (4 inches), the filter cake **154** leaves the filter or series of filters and enters a washing zone **155** where the filter cake **154** Is washed with a wash feed **149.** Pressure can then be applied to the wash feed **149** to displace the solute (i.e. the liquid and any dissolved compounds such as metal catalyst in the filter cake) of the filter cake **154.** Upon displacement of the solute with the wash feed, the filter cake **154** can be discharged from the filtration zone **155** by any suitable means, and the cycle repeated. In an embodiment, the ratio of wash feed **149** to filter cake **154** discharge Is within the range of from 1:20 to 20:1 to reduce the level of metal catalyst In the filter cake by greater than 95 wt%.

Equipment for performing the requisite washing cycle can comprise a series of filter cells maintained in a suitable position to permit a wash feed **149** reservoir to develop over the filter cells. In one embodiment of the Invention, suitable equipment can comprise a rotary drum pressure filter with multiple filter cells, fitted with a means for discharging washed cake **146** from the filter cells. The filter cake **154** can be washed for as many times as required to develop a minimum concentration of metal catalyst in the washed cake **146** before discharging the washed cake **146** from the filter device.

A suitable pressure filter which can be adapted to the requirements of the instant invented process is a BHS-FEST™ rotary drum pressure filter, BHS-WERK, Sonthofen, D-8972, Sonthofen, West Germany, although other pressure filters which can accomplish the required operation can be used. Examples of other devices that can be used in the solid-liquid separation zone include **151,** but are not limited to, pressure belt filters, filter presses, centrifuges, pressure leaf filters, and cross-flow filters. The pressure filter can be operated at a temperature and pressure sufficient to obtain at least 80 wt% recovery of the metal catalyst from the solute of the mother liquor **147.** Preferably, the pressure filter can be operated at a temperature of about 25°C to about 160 °C, and a pressure of 1x100 kPa (1 atmosphere) to 5x1000 kPa (50 atmospheres)

In the operation of the BHS-FEST™ filter, a rotary drum contains a series of filter cells located on the periphery of the rotating drum. As the drum rotates, the filter cells receive a super concentrated purge slurry **145** and a filter cake **154** builds to a requisite depth. The mother liquor **147** is produced by filtration of the super concentrated purge slurry **145.** Upon rotation of the drum, the filter cake **154** enters a washing zone **155** where a reservoir of wash feed **149** is built up over the filter cake **154** to a required depth. The applied pressure to the wash feed reservoir forces the water through the filter cake **154** to displace the solute (with dissolved metal catalyst) retained in the super concentrated purge slurry **145** to produce a washed cake **146.** Upon further rotation of the drum, the wash cycle can be repeated at least three more times if necessary in a counter current fashion, after which the system pressure is released with attendant temperature decrease to ambient conditions. Optionally, the washed cake **146** can be dewatered in a dewatering zone 157 with a vapor via conduit **152** to produce a dewatered cake **159** and a humid vapor **160.** The resultant dewatered cake **159** can then be discharged from the drum by any conventional means.

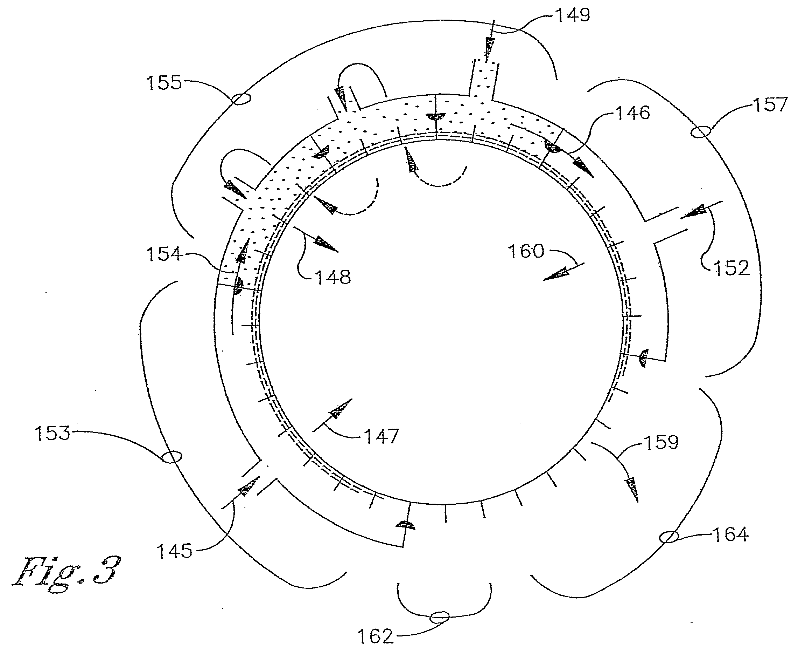

Figure 3 illustrates an embodiment where a rotary pressure drum filter is utilized as the process filtration device. In an embodiment, the rotary drum pressure filter comprises a filtration zone **153,** a wash zone **155,** optionally, a dewatering zone **157,** a discharge zone **164** and a cloth wash zone **162.** The cloth wash zone shown in Figure 3 is an embodiment where the rotary pressure drum filter comprises a cloth wash zone **162** where the filters are washed after discharge of the dewatered cake **159.**

The wash filtrate **148** is produced by displacement washing the filter cake with the wash feed **149.** The filter cake **154** within the solid-liquid separation zone **151** undergoes extraction of metal catalyst by introduction of the wash feed **149** to form the wash filtrate **148.** In an embodiment of the Invention, at least 80 wt% of the metal catalyst is recovered In the wash filtrate **148** and the mother liquor **147.** In an embodiment of the invention, at least 90 wt% of the metal catalyst is recovered in the wash filtrate **148** and the mother liquor **147.** The wash feed **149** comprises water and optionally an additional oxidation solvent.

Perhaps most surprisingly by utilizing water as a wash feed **149** at temperatures in the range of about 20°C to about 70°C, preferably about 30°C to about 50°C, sufficient corrosion metal is retained in the dewatered cake **159** wherein the need for corrosion metal removal by other means is eliminated. The dewatered cake **159** which represents solids stripped of metal catalyst can be disposed from the system.

Step (e) optionally comprises subjecting the mother liquor **147** and optionally the wash filtrate **148** to evaporation In an evaporator zone **210** to produce a solvent rich vapor stream **202** and wash filtrate residue **201.**

The evaporator zone **210** comprises at least one evaporator. In an embodiment, the evaporator is operated at atmospheric or slightly super atmospheric conditions, generally from 1x100 kPa (1 atmosphere) to 1x1000 kPa (10 atmospheres). The solvent rich vapor **202** comprises a majority of the water and solvent, and the wash filtrate residue **201** comprises the remainder of the water and solvent not removed from the mother liquor **147** and the majority of the catalyst. The evaporation removes 90 wt% to 99 wt% of the solvent and water from the combined stream in conduit **147,** typically acetic acid and water, which are present in the wash filtrate **148** and the majority of the benzoic acid in the mother liquor **147.** "Majority" as used herein means greater than 50% by weight.

Step (f) comprises subjecting the solvent rich vapor stream **202** to conventional distillation in distillation zone **220** to form a benzoic acid rich stream **203** and a solvent rich stream **204.**

The separation zone **220** comprises at least one liquid-vapor separator. In an embodiment the separator operates at atmospheric or slightly super atmospheric conditions, generally from 1x100 kPa (1 atmosphere) to 1x1000 kPa (10 atmospheres). The liquid-vapor separator comprises at least one theoretical vapor-liquid equilibrium stage. Examples of liquid-vapor separators include, but are not limited to, flash condensers and distillation columns.

In an embodiment, the benzoic rich acid stream **203** has greater than 5 wt% benzoic acid. In another embodiment, the benzoic acid rich stream **203** has greater than 15 wt% benzoic acid. In another embodiment the benzoic acid rich stream **203** has greater than 30 wt% benzoic acid. In another embodiment, the benzoic acid rich stream **203** has greater than 50 wt% benzoic acid. In another embodiment, the benzoic acid rich stream **203** comprises from about 5 wt% to 75 wt% benzoic acid. in another embodiment, the benzoic acid rich stream **203** comprises from about 5 wt% to 50 wt% benzoic acid. In another embodiment the benzoic acid rich stream **203** comprises from about 5 wt% to 35 wt% benzoic acid. In another embodiment, the benzoic acid rich stream **203** comprises from about 15 wt% to 30 wt% benzoic acid.

Step (g) comprises optionally recycling at least a portion of the solvent rich stream **204** back to an oxidation reactor in an aromatic oxidation process.

At least a portion of the solvent rich stream can be recycled back to an oxidation reactor in the oxidation process. "At least a portion" can mean at least 5 wt%, at least 15 wt%, at least 30 wt%, at least 50 wt%, at least 75 wt%, or all of the solvent rich stream is recycled **204** back to an oxidation reactor.

An example of an aromatic oxidation process is disclosed US. Patent Application 10/156,312.

Although the composition of the various streams In the process varies depending on the process conditions, a typical composition of the streams, using a computer simulated model (ASPEN version 12.1) of the process, are shown in Tables 1a and 1b. In Tables 1a and 1b, the components are shown in the left hand column, and the amount of these components in each stream In Figure 1 are shown in the number column corresponding to the number of the stream in Figure 1.

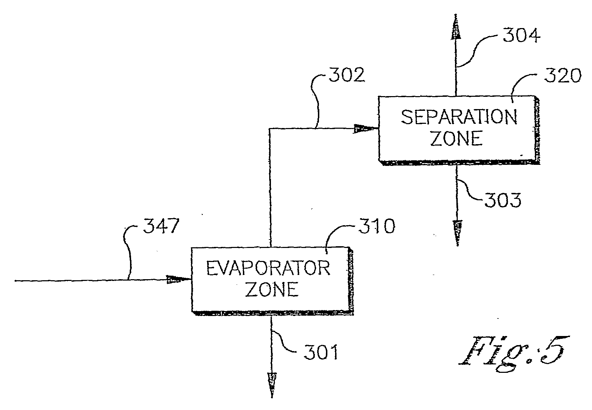

According to the invention, a process is provided as shown in Figure 5.

Step (a) comprises subjecting a benzoic acid bearing stream **347** to evaporation In an evaporator zone **310** to produce a solvent rich vapor **302** and wash filtrate residue **301**

The evaporator zone **310** comprises at least one evaporator The benzoic acid bearing stream **347** comprises water and benzoic acid. In an embodiment of the invention, the evaporator is operated at atmospheric or slightly super atmospheric conditions, generally from 1x100 kPa (1 atmosphere) to 1x1000 kPa (10 atmospheres). The solvent rich vapor **302** comprises a majority of the water and solvent, and the wash filtrate residue **301** comprises the remainder of the water and solvent not removed from the benzoic acid bearing stream **347.** In anoter embodiment of the invention, the evaporation removes about 90 wt% to about 99 wt% of the solvent and water from the benzoic acid bearing stream **347,** typically acetic acid and water.

The benzoic acid bearing stream is a stream produced in an aromatic oxidation process of para-xylene to terephthalic acid. An example of an aromatic oxidation process is disclosed in US. Patent Application 10/156,312.

For example, during the course of the oxidation reaction, exothermic heat of reaction and water generated by the oxidation of the dialkyl aromatic compound are removed from the reactor through the vaporization of a portion of the liquid reaction medium. These vapors, known as reactor offgas, comprise the aqueous solvent having five to thirty weight percent water and oxygen-depleted process gas containing minor amounts of decomposition products including catalyst residue. The reactor offgas can be used as the benzoic acid bearing stream.

In an embodiment of the invention, the evaporator zone, **310,** Is operated at atmospheric or slightly super atmospheric conditions, generally from 1x100 kPa (1 atmosphere) to 1x1000 kPa (10 atmospheres). The solvent rich vapor stream, **302,** comprises benzoic acid, and the wash filtrate residue, **301,** comprises solids not captured in the solid-liquid separation zone, including any residual catalyst metals.

Step (b) comprises subjecting the solvent rich vapor stream **302** to distillation in seperation zone **320** to form a benzoic acid rich stream **303** and a solvent rich stream **304.**

The separation zone **320** comprises at least one liquid-vapor separator. In an embodiment of the invention, the liquid vapor separator operates at atmospheric or slightly super atmospheric conditions, generally from 1x100 kPa (1 atmosphere) to 1x1000 kPa (10 atmospheres). The liquid-vapor separator comprises at least one theoretical vapor-liquid equilibrium stage. Examples of liquid-vapor separators include, but are not limited to, flash condensers and distillation columns.

The benzoic rich acid stream **303** comprises at least 5 wt% benzoic acid. In another embodiment of the invention, the benzoic acid stream has greater than 15 wt% benzoic acid.

In another embodiment of the invention, the benzoic acid rich stream **303** has greater than 30 wt% benzoic acid. In another embodiment of the invention the benzoic acid rich stream **303** has greater than 50 wt% benzoic acid. In another embodiment of the invention, the benzoic acid rich stream **303** comprises from 5 wt% to 75 wt% benzoic acid: in another embodiment of the invention, the benzoic acid rich stream **303** comprises from 5 wt% to 50 wt% benzoic acid. In another embodiment of the Invention, the benzoic acid rich stream **303** comprises from 5 wt% to 35 wt% benzoic acid. In another embodiment of the invention, the benzoic acid rich stream **303** comprises from 15 wt% to 30 wt% benzoic acid,

Step (c) comprises optionally recycling at least a portion of the solvent rich stream **304** back to an oxidation reactor in an aromatic oxidation process.

At least a potion of the solvent rich stream can be recycled to an oxidation reactor in the oxidation process. At least a portion can mean at least 5, at least 15 wt%, at least 30 wt%, at least 50 wt%, at least 75 wt% or all of the solvent rich stream is recycled **304** to an oxidation reactor.

## Claims

1. A process comprising:
(a) subjecting a benzoic acid bearing stream (347) comprising water and benzoic acid to evaporation in an evaporator zone (310) to form a solvent rich vapor (302) and a wash filtrate residue (301); and
(b) subjecting said solvent rich vapor (302) to distillation in a separation zone (320) to form a solvent rich stream (304) and a benzoic acid rich stream (303); wherein said benzoic acid rich stream (303) comprises at least 5% by weight benzoic acid;
wherein the solvent includes acetic acid and water, and wherein said benzoic acid bearing stream (347) is produced in an aromatic oxidation process of p-xylene to terephthalic acid.

2. The process according to claim 1 wherein said benzoic acid rich stream (303) comprises benzoic acid in an amount greater than 30% by weight.

3. The process according to claim 1 wherein said benzoic acid bearing stream (347) is a vapor stream produced in the oxidation of said aromatic compound.

4. The process according to claim 1 wherein said evaporator zone (310) is operated between 1 x 10⁵ Pa to 1 x 10⁶ Pa (1 to 10 atmospheres).

5. The process according to claim 1, wherein said benzoic acid rich stream (303) comprises at least 15% by weight benzoic acid; and further comprising (c) recycling at least a portion of the solvent rich stream (304) back to an oxidation reactor in said aromatic oxidation process.

6. The process according to claim 5 wherein said benzoic acid rich stream (303) comprises benzoic acid in an amount greater than 30% by weight.

7. The process according to claim 5 wherein said benzoic acid bearing stream (347) is a vapor stream produced in the oxidation of said aromatic compound.

8. The process according to claim 5 wherein at least 50 wt% of said benzoic acid rich stream (303) is recycled to an oxidation reactor in said aromatic oxidation process.

9. The process according to claim 5 wherein said evaporator zone (310) is operated between 1 x 10⁵ Pa to 1 x 10⁶ Pa (1 to 10 atmospheres).

10. The process according to claim 1, wherein said benzoic acid rich stream (303) comprises from 5 wt% to 35 wt% benzoic acid; and further comprising (c) recycling at least 50 wt% of the solvent rich stream (304) to an oxidation reactor in said aromatic oxidation process.

11. The process according to claim 10 wherein said benzoic acid bearing stream (347) is a vapor stream produced in the oxidation of said aromatic compound.

12. The process according to claim 10 wherein at least 75 wt% of said benzoic acid rich stream (303) is recycled to an oxidation reactor in said aromatic oxidation process.

13. The process according to claim 10 wherein said evaporator zone (310) is operated between 1 x 10⁵ Pa to 1 x 10⁶ Pa (1 to 10 atmospheres).

14. The process according to claim 10 wherein all of the solvent rich stream (304) is recycled to an oxidation reactor in said aromatic oxidation process.

## Patentansprüche

1. Verfahren, umfassend:
(a) Unterziehen eines benzoesäuretragenden Stroms (347), welcher Wasser und Benzoesäure umfasst, einer Verdampfung in einer Verdampferzone (310), um einen lösungsmittelreichen Dampf (302) und einen Waschfiltratrückstand (301) zu bilden; und
(b) Unterziehen des besagten lösungsmittelreichen Dampfs (302) der Destillation in einer Trennzone (320), um einen lösungsmittelreichen Strom (304) und einen benzoesäurereichen Strom (303) zu bilden; wobei besagter benzoesäurereiche Strom (303) mindestens 5 Gew.-% Benzoesäure aufweist;
wobei das Lösungsmittel Essigsäure und Wasser enthält, und wobei der besagte benzoesäuretragende Strom (347) in einem Oxidationsverfahren für Aromaten von p-Xylol zu Terephthalsäure erzeugt wird.

2. Verfahren nach Anspruch 1, wobei der besagte benzoesäurereiche Strom (303) Benzoesäure in einer Menge von mehr als 30 Gew.-% umfasst.

3. Verfahren nach Anspruch 1, wobei der besagte benzoesäuretragende Strom (347) ein Dampfstrom ist, der bei der Oxidation der besagten aromatischen Verbindung erzeugt wird.

4. Verfahren nach Anspruch 1, wobei die besagte Verdampferzone (310) bei einem Druck zwischen 1 x 10⁵ Pa bis 1 x 10⁶ Pa (1 bis 10 Atmosphären) betrieben wird.

5. Verfahren nach Anspruch 1, wobei der besagte benzoesäuretragende Strom (303) mindestens 15 Gew.-% Benzoesäure aufweist; und weiterhin umfassend (c) das Rückführen mindestens eines Teils des lösungsmittelreichen Stroms (304) zurück zu einem Oxidationsreaktor im besagten Oxidationsverfahren für Aromaten.

6. Verfahren nach Anspruch 5, wobei der besagte benzoesäurereiche Strom (303) Benzoesäure in einer Menge von mehr als 30 Gew.-% umfasst.

7. Verfahren nach Anspruch 5, wobei der besagte benzoesäuretragende Strom (347) ein Dampfstrom ist, welcher bei der Oxidation der besagten aromatischen Verbindung erzeugt wird.

8. Verfahren nach Anspruch 5, wobei mindestens 50 Gew.-% des besagten benzoesäurereichen Stroms (303) zu einem Oxidationsreaktor in besagtem Oxidationsverfahren für Aromaten zurückgeführt werden.

9. Verfahren nach Anspruch 5, wobei die besagte Verdampferzone (310) bei einem Druck zwischen 1 x 10⁵ Pa bis 1 x 10⁶ Pa (1 bis 10 Atmosphären) betrieben wird.

10. Verfahren nach Anspruch 1, wobei der besagte benzoesäurereiche Strom (303) 5 Gew.-% bis 35 Gew.-% Benzoesäure umfasst; und weiter umfassend (c) das Rückführen mindestens 50 Gew.-% des besagten lösungsmittelreichen Stroms (304) zu einem Oxidationsreaktor im besagten Oxidationsverfahren für Aromaten.

11. Verfahren nach Anspruch 10, bei dem der besagte benzoesäuretragende Strom (347) ein Dampfstrom ist, der bei der Oxidation der besagten aromatischen Verbindung erzeugt wird.

12. Verfahren nach Anspruch 10, wobei mindestens 75 Gew% des besagten benzoesäurereichen Stroms (303) zu einem Oxidationsreaktor im besagten Oxidationsverfahren für Aromaten zurückgeführt werden.

13. Verfahren nach Anspruch 10, wobei die besagte Verdampferzone (310) bei einem Druck zwischen 1 x 10⁵ Pa bis 1 x 10⁶ Pa (1 bis 10 Atmosphären) betrieben wird.

14. Verfahren nach Anspruch 10, wobei der gesamte lösungsmittelreiche Strom (304) zu einem Oxidationsreaktor im Oxidationsverfahren für Aromaten zurückgeführt wird.

## Revendications

1. Procédé consistant :
(a) à soumettre un courant portant de l'acide benzoïque (347) comprenant de l'eau et de l'acide benzoïque à une évaporation dans une zone d'évaporateur (310) pour former une vapeur riche en solvant (302) et un résidu de filtrat de lavage (301) ; et
(b) à soumettre ladite vapeur riche en solvant (302) à une distillation dans une zone de séparation (320) pour former un courant riche en solvant (304) et un courant riche en acide benzoïque (303) ; dans lequel ledit courant riche en acide benzoïque (303) comprend au moins 5 % en masse d'acide benzoïque ;
dans lequel le solvant comprend de l'acide acétique et de l'eau, et dans lequel ledit courant portant de l'acide benzoïque (347) est produit dans un procédé d'oxydation aromatique de p-xylène en acide téréphtalique.

2. Procédé selon la revendication 1, dans lequel ledit courant riche en acide benzoïque (303) comprend de l'acide benzoïque dans une quantité supérieure à 30 % en masse

3. Procédé selon la revendication 1, dans lequel ledit courant portant de l'acide benzoïque (347) est un courant de vapeur produit dans l'oxydation dudit composé aromatique.

4. Procédé selon la revendication 1, dans lequel ladite zone d'évaporateur (310) fonctionne entre 1 x 10⁵ Pa et 1 x 10⁶ Pa (1 à 10 atmosphères).

5. Procédé selon la revendication 1, dans lequel ledit courant riche en acide benzoïque (303) comprend au moins 15 % en masse d'acide benzoïque ; et comprend de plus (c) le recyclage d'au moins une portion du courant riche en solvant (304) vers un réacteur d'oxydation dans ledit procédé d'oxydation aromatique.

6. Procédé selon la revendication 5, dans lequel ledit courant riche en acide benzoïque (303) comprend de l'acide benzoïque dans une quantité supérieure à 30 % en masse.

7. Procédé selon la revendication 5, dans lequel ledit courant portant de l'acide benzoïque (347) est un courant de vapeur produit dans l'oxydation dudit composé aromatique.

8. Procédé selon la revendication 5, dans lequel au moins 50 % en masse dudit courant riche en acide benzoïque (303) sont recyclés vers un réacteur d'oxydation dans ledit procédé d'oxydation aromatique.

9. Procédé selon la revendication 5, dans lequel ladite zone d'évaporateur (310) fonctionne entre 1 x 10⁵ Pa et 1 x 10⁶ Pa (1 à 10 atmosphères).

10. Procédé selon la revendication 1, dans lequel le dit courant riche en acide benzoïque (303) comprend de 5 % en masse à 35 % en masse d'acide benzoïque; et comprend de plus (c) le recyclage d'au moins 50 % en masse du courant riche en solvant (304) vers un réacteur d'oxydation dans ledit procédé d'oxydation aromatique.

11. Procédé selon la revendication 10, dans lequel ledit courant portant de l'acide benzoïque (347) est un courant de vapeur produit dans l'oxydation dudit composé aromatique.

12. Procédé selon la revendication 10, dans lequel au moins 75 % en masse dudit courant riche en acide benzoïque (303) sont recyclés vers un réacteur d'oxydation dans ledit procédé d'oxydation aromatique.

13. Procédé selon la revendication 10, dans lequel ladite zone d'évaporateur (310) fonctionne entre 1 x 10⁵ Pa et 1 x 10⁶ Pa (1 à 10 atmosphères).

14. Procédé selon la revendication 10, dans lequel la totalité du courant riche en solvant (304) est recyclée vers un réacteur d'oxydation dans ledit procédé d'oxydation aromatique.
